(19) Europäisches Patentamt
European Patent Office
Office européen des brevets

(11) Publication number: 0 114 657
B1

(12) EUROPEAN PATENT SPECIFICATION

(45) Date of publication of patent specification: 18.03.87

(21) Application number: 84100502.8

(22) Date of filing: 18.01.84

(51) Int. Cl.$^4$: C 07 C 51/12, C 07 C 59/06, C 07 C 59/08, C 07 C 59/125, C 07 C 59/13, C 07 C 69/675, C 01 B 33/28, B 01 J 29/28

# (54) Process for production of hydroxycarboxylic acids.

(30) Priority: 20.01.83 JP 7611/83

(43) Date of publication of application: 01.08.84 Bulletin 84/31

(45) Publication of the grant of the patent: 18.03.87 Bulletin 87/12

(84) Designated Contracting States: DE GB NL

(56) References cited:
EP-A-0 031 255
EP-A-0 088 529
DE-A-3 107 518
DE-A-3 133 353

(73) Proprietor: MITSUBISHI GAS CHEMICAL COMPANY, INC.
5-2, Marunouchi 2-chome Chiyoda-Ku
Tokyo (JP)

(72) Inventor: Suzuki, Takashi
1-4224, Tsukefune-cho
Niigata-shi Niigata-ken (JP)
Inventor: Hashimoto, Shoichiro
1-13-31, Ohgata-honcho
Niigata-shi Niigata-ken (JP)
Inventor: Orisaku, Masami
1-6247-6, Igarashi
Niigata-shi Niigata-ken (JP)
Inventor: Nakano, Rieko
4-13-9, Higashi nakajima
Niigata-shi Niigata-ken (JP)

(74) Representative: Kraus, Walter, Dr. et al
Patentanwälte Kraus, Weisert & Partner
Thomas-Wimmer-Ring 15
D-8000 München 22 (DE)

EP 0 114 657 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

**Description**

This invention relates to a process for producing hydroxycarboxylic acids and/or their derivatives represented by the following formula

$$R^4O-\underset{\underset{O}{\|}}{C}-\underset{\underset{R}{|}}{CH}-OR^5$$

wherein R represents a hydrogen atom, an aliphatic hydrocarbon group, an alicyclic group or a carbocyclic aromatic group, $R^4$ represents a hydrogen atom or an aliphatic hydrocarbon group, and $R^5$ represents a hydrogen atom or an aliphatic acyl group comprising reacting an aldehyde compound, carbon monoxide and water in the presence of aluminosilicate in a reaction medium.

Hydroxycarboxylic acids are important compounds which are widely used in the chemical industry. For example, glycolic acid is directly used as a chemical detergent and is also useful as an intermediate for organic syntheses, a raw material for polymers and an additive.

Methods have previously been known for producing glycolic acid, a hydroxycarboxylic acid, from formaldehyde as a starting material. They include a method which comprises reacting formaldehyde, water and carbon monoxide under a high pressure using a mineral acid such as sulfuric acid, phosphoric acid and hydrochloric acid as a catalyst to form glycolic acid in one step (see, for example, the specifications of U.S. Patents Nos. 2,152,852 and 2,153,064 and Japanese Patent Publication No. 44,454/1978) and a method which comprises reacting formaldehyde, water and carbon monoxide in the presence of hydrogen fluoride under a high pressure to form glycolic acid in one step (see, for example, the specifications of U.S. Patents Nos. 3,754,028 and 3,911,003 and Japanese Laid-Open Patent Publication No. 13,719/1976). These methods, however, have proved to be of little use in the chemical industry because they require a complex operation for separating glycolic acid from the reaction mixture, the mineral acids and hydrogen fluoride used as catalysts are difficult to recover from the reaction mixture for re-use, and acid-resistant reactors are necessary to withstand the strong acidity of the catalysts.

The specification of Japanese Patent Publication No. 37,332/1978 discloses a method for producing hydroxycarboxylic acids which comprises reacting aliphatic aldehydes and carbon monoxide in a liquid reaction medium in the presence of a solid insoluble granular acid catalyst. As the insoluble granular acid catalyst used in this method, this patent document specifically shows clay minerals such as kaolinite, halloysite, smectite, illite, vermiculite, chlorite, sepiolite, attapulgite and palygorskite, and acid catalysts derived from montmorillonite and natural or synthetic mordenites. It does not disclose FZ-1 or ZSM-type zeolites.

Japanese Laid-Open Patent Publication No. 122,321/1981 discloses a method for producing an ether or ester of glycolic acid which comprises reacting formaldehyde, carbon monoxide and a formic acid ester in the presence of a strong acid catalyst. Examples of the strong acid catalyst which are shown in this patent document are Brφnsted acids such as mineral acids and organic acids, Lewis acids such as boron trifluoride, strongly acidic cationic exchange resins having sulfonic acid groups as functional groups, clay minerals, zeolites, solidified acids resulting from supporting of phosphoric acid or boric acid on carriers, inorganic oxides, inorganic salts and double oxides. This patent document only describes montmorillonite, kaolinite, bentonite, halloysite, smectite, illite, vermiculite, chlorite, sepiolite, attapulgite, palygorskite and mordenite as specific examples of the clay minerals and zeolite (natural zeolite).

DE—A—3,107,518 describes the use of aluminosilicates as solid insoluble acid catalysts in the process of producing hydroxycarboxylic acids and/or their derivatives through the reaction of an aldehyde compound, carbon monoxide and water in a reaction medium.

It is the object of this invention to provide a process for producing hydroxycarboxylic acids industrially advantageously by using a specified crystalline aluminosilicate catalyst which has high catalytic activity, high heat resistance (heatproof property) and a long life and is easy to handle.

The desired process for producing hydroxycarboxylic acids should be industrially advantageously, and a high conversion of the starting aldehyde compound and a high selectivity for the hydroxycarboxylic acids should be achieved.

The object and the advantages of this invention are achieved in accordance with this invention by a process as described above which is characterized in that the aluminosilicate is FZ-1 zeolite or ZSM-type zeolite whose X-ray diffraction pattern shows at least five clear diffraction lines at a diffraction angle 2θ of 7.9°±0.3°, 8.8°±0.3°, 23.2°±0.3°, 23.7°±0.3° and 24.4°±0.3° while the concentration of the aldehyde compound in the reaction mixture is maintained at not more than 15% by weight based on the total weight of the aldehyde compound and the reaction medium.

The aldehyde compound used in the process of this invention may be aliphatic, alicyclic or aromatic. Aliphatic aldehydes are commercially preferred. It may be a monoaldehyde or a polyaldehyde such as a dialdehyde.

Preferably, the aliphatic aldehyde can be represented by the following formula

$$R^1-CHO \qquad (1)$$

wherein $R^1$ represents a hydrogen atom or an aliphatic hydrocarbon group.

Preferably, the alicyclic aldehyde can be represented by the following formula

$$R^2—CHO \qquad (2)$$

wherein $R^2$ represents an alicyclic group.

Preferably, the aromatic aldehyde can be represented by the following formula

$$R^3—CHO \qquad (3)$$

wherein $R^3$ represents a carbocyclic aromatic group.

The aliphatic hydrocarbon group ($R^1$) in general formula (1) may be linear or branched, and saturated or unsaturated. Generally, aliphatic hydrocarbon groups having 1 to 10 carbon atoms are preferred, and alkyl groups having 1 to 4 carbon atoms are especially preferred. Examples of the aliphatic hydrocarbon groups include methyl, ethyl, n-propyl, iso-propyl, n-butyl and iso-butyl groups. Thus, examples of the aliphatic aldehyde include formaldehyde, acetaldehyde, propionaldehyde, n-butyraldehyde and isobutyraldehyde. Of these, formaldehyde, acetaldehyde and propionaldehyde are preferred. Formaldehyde is especially preferred. Formaldehyde may be fed into the reaction system as such or in the form of paraformaldehyde, alpha-polyoxymethylene, trioxane, tetraoxane, etc., or as formalin (preferably an aqueous solution containing at least 37% of formaldehyde).

The alicyclic group ($R^2$) in general formula (2) may be saturated or unsaturated. Preferably, the alicyclic group is a 5- or 6-membered aliphatic carbocyclic groups. Examples are cyclopentyl and cyclohexyl groups. Thus, examples of the alicyclic aldehyde are cyclopentanecarbaldehyde (formyl cyclopentane) and cyclohexanecarbaldehyde (formyl cyclohexane).

The carbocyclic aromatic group ($R^3$) in general formula (3) is preferably a benzene ring or a naphthalene ring. Examples of the aromatic aldehyde are benzaldehyde, naphthaldehyde and terephthaldehyde.

Carbon monoxide used in the reaction is not only highly pure carbon monoxide, but also carbon monoxide containing nitrogen, hydrogen, and/or carbon dioxide which do not affect the reaction.

The basic reaction in accordance with this invention can be represented by the following reaction formula when the aldehyde compound is formaldehyde.

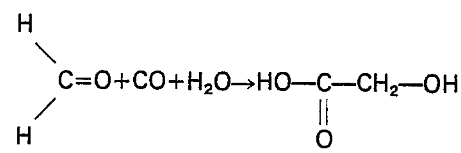

In addition to the aldehyde compound and carbon monoxide, water participates as one reactant in the reaction of this invention.

The process of this invention is carried out in the presence of a reaction medium. Preferably, the reaction medium is a neutral or acidic substance which is liquid under the reaction conditions. Examples of preferred reaction media include lower aliphatic carboxylic acids such as acetic acid, propionic acid and butyric acid, halogenated hydrocarbons such as dichloromethane, chloroform and carbon tetrachloride, esters between lower aliphatic carboxylic acids and lower aliphatic alcohols such as ethyl acetate and methyl propionate, lower aliphatic alcohols such as methanol, ethanol and propanol, water, and mixtures of these. Of these, the lower aliphatic carboxylic acids such as acetic acid, propionic acid and butyric acid are especially preferred. Water in the amount required for the reaction is usually introduced into the reaction system while it is carried, for example, by a source of the aldehyde and/or the reaction medium. Preferably, the concentration of water is not more than 30% by weight based on the total weight of the aldehyde and the reaction medium.

In the process of this invention, it is essential that the concentration of the aldehyde compound in the reaction mixture be maintained at not more than 15%, preferably 5 to 15%, especially preferably 5 to 12%, based on the total weight of the aldehyde compound and the reaction medium. By maintaining the concentration of the aldehyde compound in the reaction mixture at not more than 15%, a high conversion of the aldehyde compound and a high selectivity for the hydroxycarboxylic acid can be achieved in the reaction system of this invention.

A crystalline aluminosilicate of FZ-1 zeolite or ZSM-type zeolite is used as a catalyst in the process of this invention. The crystalline aluminosilicate catalyst is characterized by the fact that in its X-ray diffraction pattern, it shows at least five clear diffraction lines at a diffraction angle 2θ of 7.9°±0.3°, 8.8°±0.3°, 23.2°±0.3°, 23.7°±0.3° and 24.4°±0.3°.

The X-ray diffraction analysis is effected by a standard powder diffraction method using $CuK_\alpha$ radiation.

The crystalline aluminosilicates used in this invention have a specified cage structure and at least contain silicon, oxygen and aluminum atoms. Crystalline aluminosilicates which additionally contain a

metal atom of Group III of the periodic table such as boron or a transition metal atom such as chromium may be used in this invention if they are characterized by the aforesaid X-ray diffraction pattern.

Examples of suitable crystalline aluminosilicates used in the invention are FZ-1 zeolites, FZ-1 zeolites containing transition metals and ZSM-type zeolites typified by ZSM-5 zeolite, which are described, for example, in Japanese Laid-Open Patent Publications Nos. 88,820/1981 and 15,023/1983 and Japanese Patent Publication No. 18,521/1978, respectively. It should be understood that the disclosures of these patent documents about zeolite constitute part of the disclosure of the present application.

FZ-1 zeolite and ZSM-5 zeolite used as the catalyst of this invention are characterized by having clear diffraction lines at the following diffraction angles 2θ(°).

| FZ-1 | ZSM-5 |
|---|---|
| 7.9 | 7.96 |
| 8.8 | 8.84 |
| 17.7 | 23.10 |
| 23.2 | 24.00 |
| 23.7 | 24.45 |
| 23.9 | |
| 24.4 | |

The crystalline aluminosilicate used in this invention acts as an acid catalyst in the reaction system. Preferably, therefore, the crystalline aluminosilicate is used after it has been subjected to an activating treatment. For example, a crystalline aluminosilicate of the FZ-1 series is activated by calcining it, treating the calcined product with an aqueous solution of hydrogen chloride under reflux, drying it, and after optionally subjecting it to ion exchange in a customary manner, calcining it again. The ZSM-type zeolite is activated by calcining it, treating the calcined product with an aqueous solution of ammonium chloride under reflux, drying it and then calcining it.

The crystalline aluminosilicate used in the process of this invention has high catalytic activity in a reaction for producing hydroxycarboxylic acids from an aldehyde compound, carbon monoxide and water. Hence, even when it is used, for example, in an amount which provides a hydrogen ion concentration of about 0.5 millimole per mole of the aldehyde, it exhibits sufficient catalytic activity in the reaction of this invention.

Since the crystalline aluminosilicate can be easily separated and recovered from the reaction mixture after the reaction without any substantial loss, it can be caused to be present in the reaction system in a relatively large amount and the rate of the reaction can therefore be increased. According to the process of this invention, the crystalline aluminosilicate can be used in an amount of not more than 10% by weight based on the total weight of the aldehyde compound and the reaction medium in a batch system or continuous system in a suspended or fluidized bed.

The process of this invention can be carried out in any type of reactor such as a tank, packed tower, fixed bed reactor, suspended bed reactor or fluidized bed reactor. Because the crystalline aluminosilicate used in this invention has high activity in the reaction for producing hydroxycarboxylic acids, a satisfactory rate of reaction can be achieved even when the process of this invention is carried out in a continuous system which is industrially advantageous.

The reaction can be carried out usually at a temperature of 100 to 300°C. Temperatures of 180 to 250°C are especially preferred because the crystalline aluminosilicate catalyst exhibits high activity at these temperatures.

The reaction can be carried out at atmospheric pressure, but preferably under elevated pressures of usually at least 100 kg/cm$^2$.

By the basic reaction of this invention as illustrated hereinabove, a hydroxycarboxylic acid represented by the following formula

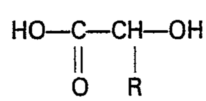

(4)

wherein R is $R^1$, $R^2$ or $R^3$, and $R^1$, $R^2$ and $R^3$ are as defined above, is formed from the aldehyde compound of the following formula

$$R{-}CHO \quad (5)$$

wherein R is as defined above, carbon monoxide and water.

R in formula (4) differs depending upon the aldehyde compound used. For example, it is a hydrogen atom for formaldehyde, a methyl group for acetaldehyde and a phenyl group for benzaldehyde.

The hydroxycarboxylic acid of formula (4) is obtained as the corresponding hydroxycarboxylic acid derivative depending upon the kind of the reaction medium used.

Investigations of the present inventors have shown that by the process of this invention, hydroxycarboxylic acids represented by the following formula

$$R^4O-\underset{\substack{\| \\ O}}{C}-\underset{\substack{| \\ R}}{CH}-OR^5 \qquad (6)$$

wherein R is as defined above, $R^4$ represents a hydrogen atom or an aliphatic hydrocarbon group, and $R^5$ represents a hydrogen atom or an aliphatic acyl group, can be produced preferably.

$R^4$ and/or $R^5$ differ depending upon the reaction medium used. For example, when methanol is used as the reaction medium, a product of formula (6) in which one of $R^4$ and $R^5$ is a methyl group and the other is a hydrogen atom or both $R^4$ and $R^5$ are methyl groups is formed. When acetic acid is used as the reaction medium, there is formed a product of formula (6) in which one of $R^4$ and $R^5$ is an acetyl group and the other is a hydrogen atom or both $R^4$ and $R^5$ are acetyl groups. Table 1 below summarizes the relation among the starting aldehyde compounds, the reaction medium and the reaction products.

TABLE 1

| Aldehyde | Reaction medium | Products |
|---|---|---|
| Formaldehyde | Water | Glycolic acid ($R^4$, $R^5$=H) |
| Formaldehyde | Acetic acid | Acetoxyacetic acid ($R^4$=H, $R^5$=acetyl); acetic acetoxyacetic anhydride ($R^4$, $R^5$=acetyl); acetic glycolic anhydride ($R^4$=acetyl, $R^5$=H) |
| Formaldehyde | Propionic acid | Propionoxyacetic acid ($R^4$=H, $R^5$=propionyl); propionic propionoxyacetic anhydride ($R^4$, $R^5$=propionyl); propionicglycolic anhydride ($R^4$=propionyl, $R^5$=H) |
| Formaldehyde | Methanol | Methoxyacetic acid ($R^4$=H, $R^5$= methyl); methyl glycolate ($R^4$=methyl, $R^5$=H); methyl methoxyglycolate ($R^4$, $R^5$=methyl) |
| Acetaldehyde | Water | Lactic acid ($R^4$, $R^5$=H) |
| Acetaldehyde | Acetic acid | Acetyllactic acid ($R^4$=H, $R^5$= acetyl); acetic acetyllactic anhydride ($R^4$, $R^5$=acetyl); acetic lactic anhydride ($R^4$=acetyl, $R^5$=H) |
| Acetaldehyde | Ethanol | alpha-Ethoxypropionic acid ($R^4$=H, $R^5$=ethyl); ethyl lactate ($R^4$=ethyl, $R^5$=H); ethyl alpha-ethoxypropionate ($R^4$, $R^5$=ethyl) |

As stated above, not only the tank reactor, but also the fixed bed reactor, continuous system and batch system can be used in practicing the present invention whereas it is not the case with the prior art. Needless to say, the continuous system is preferred in an industrial apparatus for mass production to separate and recover the catalyst and perform the operation in a steady condition.

Reactions using insoluble solid catalysts such as heteropolyacids or ion exchange resins which were recently proposed have not been studied in regard to operations in continuous-type reactors. Investigations of the present inventors have shown that the activity of these solid catalysts is much lower than that of the crystalline aluminosilicates used in the present invention, and they do not give the expected results in a continuous operation. Specifically, the present inventors have found that in the continuous

system, the reaction does not so much proceed in the presence of ion exchange resins, but the crystalline aluminosilicates specified in the present invention can give very good results of the reaction.

Since the crystalline aluminosilicate catalyst used in this invention has high heat resistance, it lends itself to easy handling and reaction operation. Furthermore, its high catalytic activity gives hydroxycarboxylic acids in good space time yields. The crystalline aluminosilicate catalyst of this invention can be used over a long period of time because it can retain its high catalytic activity over a long period of time without being attacked by the solvent. The catalyst of this invention is easy to separate and recover from the reaction mixture after the reaction, and can be re-used.

By the process of this invention, both the conversion of the aldehyde compound and the selectivity for the hydroxycarboxylic acids are increased. Moreover, besides the batch system, other systems of reaction such as a continuous system can be used in practicing the process of this invention. All of the foregoing advantages make the process of this invention very valuable in industrial practice.

The following examples illustrate the present invention more specifically.

Example 1

A 100 ml. stainless steel shaking autoclave was charged with 2 g of trioxane, 20 g of acetic acid (water content 0.1% by weight) and 2 g of an activated crystalline aluminosilicate of the ZSM-5 zeolite having a silica/alumina mole ratio of about 50, and carbon monoxide was introduced into it to a pressure of 150 kg/cm$^2$. While maintaining the temperature of the inside of the reactor at 200°C, the reaction was carried out for 3 hours.

The reaction mixture was gas-chromatographed to identify and analyze the products. It was found that the conversion of formaldehyde was 97%, and acetoxyacetic acid was obtained as a main product in a yield of 94% (selectivity 97%) based on formaldehyde. Methyl acetate (yield 2%; selectivity 2%) and traces of methyl acetoxyacetate and acetic acetoxyacetic anhydride were obtained as by-products.

Comparative Example 1

Example 1 was repeated except that the amount of trioxane charged was changed to 8.58 g. The conversion of formaldehyde was 56%. Acetoxyacetic acid, methyl acetoxyacetate and acetic acetoxyacetic anhydride were obtained in a yield, based on formaldehyde, of 51% (selectivity 91%), 3.8% (selectivity 7%), and 0.5% (selectivity 0.9%), respectively.

Examples 2 to 5

A 100 ml. stainless steel shaking autoclave was charged with 2 g (as formaldehyde) of a source of formaldehyde, 20 g of acetic acid (water content 0.1% by weight) and 2 g of each of the activated acid-type crystalline aluminosilicates shown in Table 2-a. Carbon monoxide was introduced into the autoclave to each of the pressures indicated in Table 2-a, and the reaction was carried out for 3 hours at each of the temperatures indicated in Table 2-a. The results are shown in Table 2-b.

In Examples 2, 4 and 5, 86% paraformaldehyde (the remainder being water) was used as the source of formaldehyde, and in Example 3, trioxane was used.

The crystalline aluminosilicates were activated in the following manner prior to use. ZSM-5 was calcined at 550°C for 5 hours, treated with a 10% aqueous solution of ammonium chloride under reflux for 6 hours, dried at 110°C and further calcined at 550°C for 4 hours. FZ-1 was calcined at 550°C for 5 hours, treated with an 18% aqueous solution of hydrogen chloride under reflux for 6 hours, dried at 110°C and further calcined at 550°C for 4 hours.

In Table 2-b, the yields of the products were calculated on the basis of the charged formaldehyde, and the selectivities, on the basis of the converted formaldehyde.

TABLE 2-a

| | Crystalline aluminosilicate | | Reaction conditions | | |
|---|---|---|---|---|---|
| Example | Kind | Silica/alumina mole ratio | Time (hr) | Temperature (°C) | Initial pressure of CO (kg/cm$^2$) |
| 2 | ZSM-5 | 50 | 3 | 200 | 150 |
| 3 | ZSM-5 | 50 | 3 | 200 | 100 |
| 4 | ZSM-5 | 50 | 3 | 220 | 150 |
| 5 | FZ-1 | 100 | 3 | 200 | 150 |

TABLE 2-b

| Example | Conversion of formaldehyde (%) | Yields of the products (%; the parenthesized figures show selectivities) | | |
|---|---|---|---|---|
| | | Acetoxyacetic acid | Methyl acetoxyacetate | Acetic acetoxyacetic anhydride |
| 2 | 83 | 81 (98) | trace | trace |
| 3 | 62 | 61 (98) | trace | trace |
| 4 | 67 | 66 (88) | trace | trace |
| 5 | 63 | 59 (94) | 2.6 | 0.6 |

Examples 6 to 9

ZSM-5 zeolite molded in a shape (1 mm ϕ×1 mmH) and having a silica/alumina ratio of 50, after activation by a customary method, was packed into a single tube reactor (inside diameter 10.5 mm, length 50 cm) made of Hastelloy C, and carbon monoxide was introduced into it to a pressure of 200 kg/cm$^2$. The reactor was maintained at 200°C. An acetic acid solution (starting solution; water content 0.1% by weight) of trioxane in a trioxane concentration of 9.5% by weight and carbon monoxide gas (starting gas: 92% by volume of carbon monoxide and small amounts of hydrogen, carbon dioxide and methane) were fed into the reactor at a WHSV (weight hourly space velocity) of 2.0 to 6.0 g/g-catalyst. hr. and a GHSV (gas hourly space velocity) of 0.5 to 2.0 liters/g-catalyst. hr., respectively. In Examples 6 to 8, the starting solution and the starting gas were fed as parallel downward flows, and in Example 9, they were fed as parallel upward flows. The reaction was carried out under the conditions shown in Table 3-a. The results are shown in Table 3-b.

In each run, the unreacted formaldehyde was present in the reaction mixture, and only a trace of a condensate of glycolic acid existed.

The yields and selectivities given in Table 3-b are the same as defined in Example 2. WHSV, GHSV and the space time yield (STY) of the glycolic acid derivatives are defined as follows:—

$$\text{WHSV} = \frac{\text{Amount (g) of the starting solution fed}}{\text{Amount (g) of the catalyst} \times \text{reaction time (hr)}}$$

$$\text{GHSV} = \frac{\text{Amount (liters) of the starting gas fed}}{\text{Amount (g) of the catalyst} \times \text{reaction time (hr)}}$$

$$\text{STY} = \frac{\text{Acetoxyacetic acid (g)} + \text{methyl acetoxyacetate (g)}}{\text{Amount (g) of the catalyst} \times \text{reaction time (hr)}}$$

TABLE 3-a

| Example | Reaction time (hr) | Starting solution: Amount fed (g) | Starting solution: WHSV | Starting gas: Amount fed (liter) | Starting gas: GHSV | Gas/ solution ratio (liter/g) |
|---|---|---|---|---|---|---|
| 6 | 3 | 78.1 | 2.1 | 105.8 | 2.9 | 1.47 |
| 7 | 7 | 294.5 | 2.7 | 249.6 | 2.8 | 0.92 |
| 8 | 2 | 89.3 | 2.0 | 115.6 | 3.5 | 1.41 |
| 9 | 8 | 224.3 | 2.1 | 63.4 | 0.6 | 0.31 |

TABLE 3-b

| Example | Conversion of formaldehyde (%) | Yields of the products (%) (selectivities, %): Methyl acetate | Methyl acetoxyacetate | Acetoxyacetic acid | STY of glycolic acid derivative |
|---|---|---|---|---|---|
| 6 | 82 | 1.0 (1.2) | 0.5 (0.6) | 80 (98) | 0.62 |
| 7 | 82 | 0.8 (1.0) | 0.5 (0.6) | 80 (98) | 0.83 |
| 8 | 76 | 0.8 (1.0) | 0.4 (0.5) | 74 (98) | 0.78 |
| 9 | 66 | 3.6 (5.6) | 0.4 (0.6) | 62 (94) | 0.23 |

Comparative Example 2

Example 7 was repeated except that Amberlite (a registered trademark, a product of Rohm & Haas Co.; acid type 200C, strongly acidic cation exchange resin) was used as the catalyst, and the reaction temperature was changed to 120°C.

Analysis of the reaction mixture showed that the conversion of formaldehyde was 35%, and the yield of acetoxyacetic acid based on formaldehyde was 34% (selectivity 97%).

**Claims**

1. A process for producing hydroxycarboxylic acids and/or their derivatives represented by the following formula

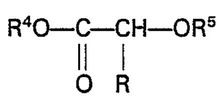

wherein R represents a hydrogen atom, an aliphatic hydrocarbon group, an alicyclic group or a carbocyclic aromatic group, $R^4$ represents a hydrogen atom or an aliphatic hydrocarbon group, and $R^5$ represents a hydrogen atom or an aliphatic acyl group comprising reacting an aldehyde compound, carbon monoxide and water in the presence of aluminosilicate in a reaction medium, characterized in that the aluminosilicate is FZ-1 zeolite or ZSM-type zeolite whose X-ray diffraction pattern shows at least five clear diffraction lines at a diffraction angle 2θ of 7.9°±0.3°, 8.8°±0.3°, 23.2°±0.3°, 23.7°±0.3° and 24.4°±0.3° while the concentration of the aldehyde compound in the reaction mixture is maintained at not more than 15% by weight based on the total weight of the aldehyde compound and the reaction medium.

2. The process of claim 1 wherein the aldehyde compound is an aliphatic aldehyde, an alicyclic aldehyde or an aromatic aldehyde.

3. The process of claim 2 wherein the aliphatic aldehyde is represented by the following formula

$$R^1—CHO$$

wherein $R^1$ represents a hydrogen atom or an aliphatic hydrocarbon group.

4. The process of claim 2 wherein the alicyclic aldehyde is represented by the following formula

$$R^2—CHO$$

wherein $R^2$ represents an alicyclic group.

5. The process of claim 2 wherein the aromatic aldehyde is represented by the following formula

$$R^3—CHO$$

wherein $R^3$ represents a carbocyclic aromatic group.

6. The process of claim 1 wherein the reaction medium is a neutral or acidic medium which is liquid under the reaction conditions.

7. The process of claim 6 wherein the reaction medium is a lower aliphatic carboxylic acid, a halogenated hydrocarbon, a lower aliphatic alcohol or an ester between a lower aliphatic carboxylic acid and a lower aliphatic alcohol.

8. The process of claim 1 wherein the reaction medium is acetic acid or propionic acid.

9. The process of claim 1 wherein the reaction temperature is 100 to 300°C.

10. The process of claim 1 wherein the concentration of the aldehyde in the reaction mixture is maintained at 5 to 15% by weight based on the total weight of the aldehyde and the reaction medium.

11. The process of claim 1 wherein the concentration of the aldehyde compound in the reaction mixture is maintained at 5 to 12% by weight based on the total weight of the aldehyde compound and the reaction medium.

12. The process of claim 1 wherein the crystalline aluminosilicate is ZSM-5 zeolite.

13. The process of claim 1 wherein the crystalline aluminosilicate is activated FZ-1 zeolite obtained by calcining FZ-1 zeolite, treating it with an aqueous solution of hydrogen chloride under reflux and then drying the treated product.

14. The process of claim 1 wherein the crystalline aluminosilicate is activated FZ-1 zeolite obtained by calcining FZ-1 zeolite, treating it with an aqueous solution of hydrogen chloride under reflux, drying the treated product, subjecting it to ion exchange treatment and again calcining the product.

15. The process of claim 1 wherein the crystalline aluminosilicate is an activated ZSM-type zeolite obtained by calcining a ZSM-type zeolite, treating it with an aqueous solution of ammonium chloride under reflux, drying the treated product and calcining it again.

**Patentansprüche**

1. Verfahren zur Herstellung von Hydroxycarbonsäuren und/oder ihrer Derivate der folgenden Formel

$$R^4O—\underset{\underset{O}{\|}}{C}—\underset{\underset{R}{|}}{CH}—OR^5$$

worin R für ein Wasserstoffatom, eine aliphatische Kohlenwasserstoffgruppe, eine alicyclische Gruppe oder eine carbocyclische aromatische Gruppe steht, $R^4$ für ein Wasserstoffatom oder eine aliphatische Kohlenwasserstoffgruppe steht und $R^5$ für ein Wasserstoffatom oder eine aliphatische Acylgruppe steht, durch Umsetzung einer Aldehydverbindung von Kohlenmonoxid und Wasser in Gegenwart von Aluminosilicat in einem Reaktionsmedium, dadurch gekennzeichnet, daß das Aluminosilicat ein FZ-1-Zeolith oder Zeolith vom ZSM-Typ ist, dessen Röntgenbeugungsmuster mindestens fünf klare Beugungslinien bei einem Beugungswinkel 2θ von 7,9°±0,3°, 8,8°±0,3°, 23,2°±0,3°, 23,7°±0,3° und 24,4°±0,3° zeigt, wobei die Konzentration der Aldehydverbindung in dem Reaktionsgemisch bei nicht mehr als 15 Gew.-%, bezogen auf das Gesamtgewicht der Aldehydverbindung und des Reaktionsmediums, gehalten wird.

2. Verfahren nach Anspruch 1, dadurch gekennzeichnet, daß die Aldehydverbindung ein aliphatischer Aldehyd, ein alicyclischer Aldehyd oder ein aromatischer Aldehyd ist.

3. Verfahren nach Anspruch 2, dadurch gekennzeichnet, daß der aliphatische Aldehyd durch die folgende Formel

$$R^1—CHO$$

angegeben wird, worin $R^1$ für ein Wasserstoffatom oder eine aliphatische Kohlenwasserstoffgruppe steht.

4. Verfahren nach Anspruch 2, dadurch gekennzeichnet, daß der alicyclische Aldehyd durch die folgende Formel

$$R^2—CHO$$

angegeben wird, worin $R^2$ für eine alicyclische Gruppe steht.

5. Verfahren nach Anspruch 2, dadurch gekennzeichnet, daß der aromatische Aldehyd durch die folgende Formel

$$R^3—CHO$$

angegeben wird, worin $R^3$ für eine carbocyclische aromatische Gruppe steht.

6. Verfahren nach Anspruch 1, dadurch gekennzeichnet, daß das Reaktionsmedium ein neutrales oder saures Medium ist, das bei Reaktionsbedingungen flüssig ist.

7. Verfahren nach Anspruch 6, dadurch gekennzeichnet, daß das Reaktionsmedium eine niedere aliphatische Carbonsäure, ein halogenierter Kohlenwasserstoff, ein niederer aliphatischer Alkohol oder ein Ester aus einer niederen aliphatischen Carbonsäure und einem niederen aliphatischen Alkohol ist.

8. Verfahren nach Anspruch 1, dadurch gekennzeichnet, daß das Reaktionsmedium Essigsäure oder Propionsäure ist.

9. Verfahren nach Anspruch 1, dadurch gekennzeichnet, daß die Reaktionstemperatur 100 bis 300°C beträgt.

10. Verfahren nach Anspruch 1, dadurch gekennzeichnet, daß die Konzentration des Aldehyds in dem Reaktionsgemisch bei 5 bis 15 Gew.-%, bezogen auf das Gesamtgewicht des Aldehyds und des Reaktionsmediums, gehalten wird.

11. Verfahren nach Anspruch 1, dadurch gekennzeichnet, daß die Konzentration des Aldehyds in dem Reaktionsgemisch bei 5 bis 12 Gew.-% bezogen auf das Gesamtgewicht der Aldehydverbindung und des Reaktionsmediums, gehalten wird.

12. Verfahren nach Anspruch 1, dadurch gekennzeichnet, daß das kristalline Aluminosilicat ZSM-5-Zeolith ist.

13. Verfahren nach Anspruch 1, dadurch gekennzeichnet, daß das kristalline Aluminosilicat aktivierter FZ-1-Zeolith ist, der dadurch erhalten worden ist, daß FZ-1-Zeolith calciniert worden ist, mit einer wäßrigen Lösung von Chlorwasserstoff unter Rückfluß behandelt worden ist und sodann das behandelte Produkt getrocknet worden ist.

14. Verfahren nach Anspruch 1, dadurch gekennzeichnet, daß das kristalline Aluminosilicat aktivierter FZ-1-Zeolith ist, der dadurch erhalten worden ist, daß FZ-1-Zeolith calciniert worden ist, mit einer wäßrigen Lösung von Chlorwasserstoff unter Rückfluß behandelt worden ist, das behandelte Produkt getrocknet worden ist, einer Ionenaustauscherbehandlung unterworfen worden ist und das Produkt erneut calciniert worden ist.

15. Verfahren nach Anspruch 1, dadurch gekennzeichnet, daß das kristalline Aluminosilicat ein aktivierter Zeolith vom ZSM-Typ ist, der dadurch erhalten worden ist, daß ein Zeolith vom ZSM-Typ calciniert worden ist, mit einer wäßrigen Lösung von Ammoniumchlorid unter Rückfluß behandelt worden ist, das behandelte Produkt getrocknet worden ist und erneut calciniert worden ist.

**Revendications**

1. Un procédé de fabrication d'acides hydroxycarboxyliques et/ou de leurs dérivés, de formule générale

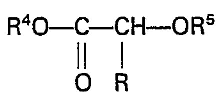

(dans laquelle R représente un atome d'hydrogène, un groupe hydrocarboné aliphatique, un groupe alicyclique ou un groupe aromatique carbocyclique, $R^4$ un atome d'hydrogène ou un groupe hydrocarboné aliphatique et $R^5$ un atome d'hydrogène ou un groupe acyle aliphatique), comprenant la réaction d'un aldéhyde avec du monoxyde de carbone et de l'eau en présence d'un alumino-silicate dans un milieu réactionnel, procédé caractérisé en ce que l'alumino-silicate est de la zéolite FZ-1 ou une zéolite du type ZSM, dont le spectre de diffraction de rayons X présente au moins cinq raies de diffraction nettes aux angles de diffraction 2θ de 7,9°±0,3°, 8,8°±0,3°, 23,2°±0,3°, 23,7°±0,3° et 24,4°±0,3°, la concentration de l'aldéhyde dans le mélange réactionnel étant maintenue au maximum à 15% du poids total de l'aldéhyde et du milieu réactionnel.

2. Le procédé selon la revendication 1, dans lequel l'aldéhyde est un aldéhyde aliphatique, alicyclique ou aromatique.

3. Le procédé selon la revendication 2, dans lequel l'aldéhyde aliphatique a la formule

$R^1$—CHO

$R^1$ étant un atome d'hydrogène ou un groupe hydrocarboné aliphatique.

4. Procédé selon la revendication 2, dans lequel l'aldéhyde alicyclique a la formule

$R^2$—CHO

$R^2$ étant un groupe alicyclique.

5. Le procédé selon la revendication 2, dans lequel l'aldéhyde aromatique a la formule

$R^3$—CHO

$R^3$ étant un groupe aromatique carbocyclique.

6. Le procédé selon la revendication 1, dans lequel le milieu réactionnel est un milieu neutre ou acide, liquide dans les conditions de la réaction.

7. Le procédé selon la revendication 6, dans lequel le milieu réactionnel est un acide carboxylique aliphatique inférieur, un hydrocarbure halogéné, un alcool aliphatique inférieur ou un ester d'un acide carboxylique aliphatique inférieur et d'un alcool aliphatique inférieur.

8. Le procédé selon la revendication 1, dans lequel le milieu de réaction est de l'acide acétique ou de l'acide propionique.

9. Le procédé selon la revendication 1, dans lequel la réaction est effectuée à une température de 100 à 300°C.

10. Le procédé selon la revendication 1, dans lequel la concentration en aldéhyde du mélange réactionnel est maintenue entre 5 et 15% du poids total de l'aldéhyde et du milieu de réaction.

11. Le procédé selon la revendication 1, dans lequel la concentration de l'aldéhyde dans le mélange réactionnel est maintenue entre 5 et 12% du poids total de l'aldéhyde et du milieu de réaction.

12. Le procédé selon la revendication 1, dans lequel l'alumino-silicate cristallisé est la zéolite ZSM-5.

13. Le procédé selon la revendication 1, dans lequel l'alumino-silicate cristallisé est une zéolite FZ-1 activée obtenue par calcination de zéolite FZ-1 puis traitement avec une solution aqueuse de chlorure d'hydrogène sous reflux et séchage du produit ainsi traité.

14. Le procédé selon la revendication 1, dans lequel l'alumino-silicate cristallisé est une zéolite FZ-1 activée obtenue par calcination de zéolite FZ-1, traitement de celle-ci avec une solution aqueuse de chlorure d'hydrogène sous reflux, séchage du produit traité, puis on soumet celui-ci à un traitement d'échange d'ions et on le calcine de nouveau.

15. Le procédé selon la revendication 1, dans lequel l'alumino-silicate cristallisé est une zéolite de type ZSM activée obtenue par calcination d'une zéolite ZSM suivie d'un traitement avec une solution aqueuse de chlorure d'hydrogène sous reflux, séchage du produit traité et à nouveau calcination de celui-ci.